Europäisches Patentamt

European Patent Office

Office européen des brevets

(11) Publication number: **0 297 292 A2**

## EUROPEAN PATENT APPLICATION

(21) Application number: 88108646.6

(51) Int. Cl.⁴: **G01N 33/52 , G01N 33/543**

(22) Date of filing: 31.05.88

(30) Priority: **10.06.87 US 60750**

(43) Date of publication of application:
**04.01.89 Bulletin 89/01**

(84) Designated Contracting States:
**AT BE CH DE ES FR GB IT LI LU NL SE**

(71) Applicant: **MILES INC.**
**1127 Myrtle Street**
**Elkhart,IN 46515(US)**

(72) Inventor: **Mickelson, Kenneth E.**
**54076 Eastwood Drive**
**Elkhart, IN 46514(US)**
Inventor: **Morris, David L.**
**23274 Ronda Drive**
**Elkhart, IN 46514(US)**
Inventor: **Motsenbocker, Marvin**
**10180 Viking Drive**
**Eden Prairie, MN 55 344(US)**

(74) Representative: **Dänner, Klaus, Dr. et al**
**c/o Bayer AG Konzernverwaltung RP**
**Patentabteilung**
**D-5090 Leverkusen 1 Bayerwerk(DE)**

(54) **Method and test device for separating labeled reagent in an immunometric binding assay.**

(57) A method for the separation of the bound and free species of a labeled reagent in an immunometric binding assay involving binding reactions in a liquid reaction mixture among analyte from a liquid test medium, a reagent particle comprising the analyte or a binding analog thereof immobilized to an insoluble particle, and a labeled reagent comprising a specific binding partner for the analyte labeled with a detectable chemical group. Analyte from the test medium binds to the labeled reagent and any of the labeled reagent not so bound binds to the reagent particle which are separated on a reagent particle separation and detection test device comprising a separation zone which is substantially impervious to the reagent particle and a detection zone for receiving the analyte bound to the labeled reagent. The reaction mixture is applied to the separation zone whereby the reagent particle is retained by the separation zone and the analyte bound to the labeled reagent is transported into the detection zone where the detectable chemical group thereof provides a detectable signal which is measured and correlated to the amount of analyte in the liquid test medium.

# METHOD AND TEST DEVICE FOR SEPARATING LABELED REAGENT IN AN IMMUNOMETRIC BINDING ASSAY

## BACKGROUND OF THE INVENTION

The present invention relates to specific binding assays which require the separation of free and bound species of a labeled reagent for the determination of an analyte in a liquid test medium. In particular, the present invention relates to heterogeneous immunoassays employing reagent particles for the separation of the free species of a labeled reagent from the bound species of a labeled reagent.

Various heterogeneous specific binding assays have been developed which generally involve specific binding interactions between the analyte to be detected, a specific binding partner for the analyte, and a labeled reagent which can be the same or different as the binding partner for the analyte. When performing such assays, the labeled reagent becomes bound to its corresponding binding partner to generate a bound species of the labeled reagent, any of the labeled reagent not so bound being the free species, wherein the extent of binding is a function of the amount of analyte present. However, since the detectable response of the labeled reagent is essentially indistinguishable in the bound and the free species, it is therefore necessary to physically separate such bound and free species from each other in order to effectively determine the amount of analyte present. Once the bound and free species of the labeled reagent have been separated from each other, the amount of label present in either fraction is determined by measuring the activity of the label and correlating such activity to the amount of analyte present.

Typically, such separation is accomplished by employing particulate solid phase reagent materials which are capable of binding to the free species in order to physically separate the free species from the bound species, but which nevertheless requires cumbersome and often inconvenient manipulative steps to effect such separation. For example, U.S. Patent No. 4,200,436 describes an immunoassay employing an immobilized form of an antigen to be measured which is added to an immunoassay reaction mixture for binding to the free species of the labeled reagent. However, in order to separate the free species bound to the immobilized antigen from the bound species, the reaction mixture must be centrifuged to effect such separation. Furthermore, once the reaction mixture has been centrifuged, either an aliquot must be obtained from the supernatant, or the supernatant must be removed, in order to measure the label of the bound species contained in the supernatant or the label of the sedimented free species, respectively.

Various methods have also been described employing particulate solid phase materials which are either immobile on a chromatographic test device, or which can be rendered immobile on such device, for the separation of the free species of the labeled reagent from the bound species. For example, European Patent Application Publication No. 120,602 describes an assay system employing a labeled reagent present partly in a form which is mobile on a selected chromatographic medium and partly in a form which is immobile. An antibody to the analyte is attached or immobilized to a particulate solid phase material which is immobile on the selected chromatographic medium and which binds to sample analyte. A labeled form of the antibody to the analyte is employed which binds to vacant binding sites of the sample analyte bound to the immobilized antibody. Any of the labeled antibody not so bound is caused to migrate away from the point of application on the chromatographic medium by applying a solvent thereto whereby the amount of label of the immobile labeled antibody bound to the solid phase material or the free labeled antibody is measured and related to the amount of analyte in the sample.

European Patent Application Publication No. 120,602 further describes the separation of agglutinated particles from nonagglutinated particles in an assay system employing latex particles as the particulate solid phase material. Depending upon the particular immunoassay format followed, e.g., competitive immunoassay or sandwich immunoassay, the latex particles agglutinate in the absence or presence, respectively, of analyte, whereby the agglutinated particles are separated from nonagglutinated particles on a selected chromatographic medium on which the former are immobile and the latter are mobile and the amount of label of the labeled reagent of the one or the other is measured.

The concentration of agglutinated particles to be measured on a bibulous support is also described by European Patent Application Publication No. 135,324 which provides an observable signal in relation to the concentration of particles on the support. In the presence of analyte, the particles employed can be inhibited from migrating or permitted to migrate, and the presence of a detectable signal at a localized or concentrated site on the support can be related to the presence of analyte. The concentration of particles is achieved by providing specific binding members as bridges between the particles. For example, in an assay for a hapten, the hapten is coupled to colored beads, which, individually, are mobile on the support, and immobile when agglutinated. The sample hapten is reacted with antibodies thereto, and then reacted with the beads for binding to available binding sites to the hapten on the beads wherein, for example, in the absence of sample hapten, there is a substantial amount of bridging between the beads by the antibodies, i.e., agglutination. Upon contact with the support, a concentration of agglutinated beads, indicated by the color of the beads, is observed in the absence of hapten as a result of the immobility thereof on the support, whereas in the presence of hapten, there is no agglutination and therefore no concentration of beads and no observable color. Similarly, for the determination of antigen, beads labeled with antibodies to the antigen and with enzymes are incubated with the sample antigen wherein bridges are formed between the beads by the antigen binding to the antibodies of the beads. The presence of antigen results in agglutination of the beads as heretofore described, and the support is contacted with a substrate for the enzyme and an appropriate indicator compound to indicate the presence of the enzyme bound to the antigen-agglutinated beads retained by the support at the site of concentration.

A method for the detection and determination of the concentration of sample analyte by agglutination or agglutination inhibition is described by German Offenlegungsschrift No. 3,511,012. The method employs a particle comprising at least one component of a bioaffinity binding system, e.g., antigen or antibody, and at least one component of a signal producing system, e.g., an enzyme, which are used in an agglutination or agglutination inhibition assay as heretofore described and as known in the art. The separation of agglutinated and nonagglutinated particles is carried out on a test device comprising an application zone, a reaction zone, a separation zone and a detection zone. The particles are either incorporated into the reaction zone or applied thereto prior to or simultaneously with the application of the sample antibody or antigen to the application zone. The presence of sample antibody or antigen either produces or prevents agglutination of the particles, respectively. The separation is accomplished by the separation zone which is permeable to the nonagglutinated particles but impermeable to the agglutinated particles. The detection zone is incorporated with another component of the signal producing system, e.g., a substrate for the enzyme, whereby the enzyme label of the nonagglutinated particles which migrate therein react with the substrate to provide a signal which is correlated to the amount of sample antigen or antibody.

One of the disadvantages of performing such competitive and two-site sandwich immunoassays where the labeled reagent bound to the solid phase material is measured, is the necessity to remove any of the labeled reagent not so bound which, if present, would otherwise result in an interfering background signal and reduced assay sensitivity. Accordingly, when performing such immunoassays employing a test device for the separation of the bound and free species of a labeled reagent, an additional wash step, employing a wash solution, is necessary to cause any of the mobile labeled reagent to migrate away from the site of the immobile labeled reagent. Furthermore, where an enzyme is employed as the label of the labeled reagent, a soluble enzyme substrate incorporated into the device would be solubilized and washed away by the wash solution and, accordingly, would therefore require either an additional step of applying an enzyme substrate solution subsequent to the wash solution, or incorporating the enzyme substrate into the wash solution.

Where the mobile species of the labeled reagent is instead measured according to such competitive and sandwich immunoassay formats, the precise measurement thereof requires that the amount of labeled reagent employed in the immunoassay be carefully controlled. Furthermore, such excess amount is limited by the need to have a signal in a measurable range. Such measurement will also result in a dose response having a high background signal in the absence of analyte.

Furthermore, in a competitive immunoassay format, the amount of antibody immobilized to the solid phase material is also critical to the performance of the immunoassay, the dose response is not linear over a wide range of analyte concentration and difficult to linearize for a narrow range, and requires extended periods of incubation.

Still further, such methods which depend upon the agglutination of particles for the separation of the bound and free species of a labeled reagent require aggregates of only a few particles to be separated from monodisperse particles, where the difference in size thereof is one order of magnitude or less. Accordingly, the choice of the chromatographic matrix must be carefully made in order to distinguish agglutinated particles from nonagglutinated particles. Such methods also require that the particles remain monodisperse prior to performance of the particular assay, as well as during the performance of an immunoassay in the absence of analyte. In particular, the agglutination of such particles can nevertheless occur, for example, during periods of storage or in the presence of biological fluids, respectively.

Accordingly, it is an object of the present invention to provide a convenient method for the rapid separation and detection of the bound species of a labeled reagent on a chromatographic test device which does not require a wash step or other cumbersome manipulative steps as heretofore described.

3

It is a further object of the present invention to provide a method for the specific binding assay determination of analyte on a chromatographic test device which does not require additional reagents or developer solutions to provide a detectable signal thereon.

Another object of the present invention is to provide a method for the specific binding assay determination of analyte on a chromatographic test device which provides a detectable signal which results in a linear dose response with little or no interfering background signal.

## SUMMARY OF THE INVENTION

The present invention provides a method for the separation of the bound and free species of a labeled reagent in an immunometric binding assay employing a reagent particle comprising the analyte or a binding analog thereof immobilized to an insoluble particle and a labeled reagent comprising a specific binding partner for the analyte labeled with a detectable chemical group. The bound and free species of the labeled reagent are separated on a reagent particle separation and detection test device comprising a solid, porous matrix which is capable of transporting aqueous liquids by capillary action. The porous matrix includes a separation zone which is substantially impervious to the reagent particle, and a detection zone which is situated a predetermined distance away from and in liquid transport contact with the separation zone. The separation and detection zones are physically distinct portions which can be located along the length of an elongate strip composed of a single, continuous, solid, porous matrix material which is substantially impervious to the reagent particle, or the physically distinct portions comprising the separation and detection zones can be composed of physically distinct solid, porous matrix materials which can be either located along the length of the elongate strip or laminated in the form of layers.

According to the method of the present invention, the liquid test medium containing the analyte, the labeled reagent, and the reagent particle are simultaneously or sequentially combined, preferably sequentially, to form an aqueous liquid reaction mixture whereby the labeled reagent binds to the analyte to form the bound species thereof, and the reagent particle is present in an amount in excess of that which is capable of binding to substantially all of the free species of the labeled reagent which does not bind to the analyte. An aliquot of the reaction mixture is then applied to the separation zone of the test device whereby the reagent particle having the free species of the labeled reagent immobilized thereto is retained and thereby prevented from migrating out of the separation zone, and the bound species of the labeled reagent is transported by the liquid test medium from the separation zone into the detection zone. The detectable chemical group of the bound species in the detection zone is then measured and correlated to the amount of analyte present in the test medium.

Since the method of the present invention does not require a wash step to cause the bound species to migrate from the separation zone into the detection zone, additional steps are not required to effect the separation thereof once the aliquot of the reaction mixture is applied to the separation zone.

Furthermore, the separation of the bound species from the immobilized free species of the labeled reagent commences essentially simultaneously with the initial generation of the detectable signal in the detection zone without additional manipulative or mechanical steps. Accordingly, the controlled separation and detection of the bound species of the labeled reagent during a substantially short period of time is provided.

Still further, since the immunometric assay method employed according to the present invention does not require a wash step to remove excess and/or nonspecifically bound labeled reagent, a soluble form of a detectant component for the label of the labeled reagent, such as a substrate for an enzyme label, can be incorporated into the test device.

## BRIEF DESCRIPTION OF THE DRAWINGS

Fig. 1 is a perspective view of a test device of the present invention comprising a separation zone and a detection zone located at physically distinct portions along the length of an elongate strip.

Fig. 2 is a perspective view of a test device of the present invention comprising a separation zone and a detection zone composed of physically distinct matrix materials in liquid transport contact located along the length of an elongate strip.

Fig. 3 is a perspective view of a test device of the present invention comprising a separation zone and a detection zone composed of physically distinct matrix materials in the form of layers in liquid transport contact.

## DESCRIPTION OF THE PREFERRED EMBODIMENTS

The method of the present invention provides an immunometric technique for the specific binding assay determination of an analyte in an aqueous liquid test medium involving binding among the analyte from the liquid test medium, the labeled reagent, and the reagent particle. Any of the labeled reagent which does not bind to the analyte from the test medium becomes bound to the analyte or binding analog thereof immobilized to the reagent particle. In carrying out the method of the present invention, the aqueous liquid reaction mixture is first formed comprising the liquid test medium, the labeled reagent, and the reagent particle, and then applied to the separation zone of the reagent particle separation and detection test device of the present invention. The reagent particle is present in an amount in excess of that which is capable of binding to all of the free species of the labeled reagent which does not become bound to the analyte from the test medium. Accordingly, the excess amount of the reagent particle is at an excess amount such that a sufficient amount is present to bind substantially all of the free species of the labeled reagent for the ultimate immobilization and separation of substantially all of the free species from the bound species.

Preferably, the labeled reagent is also present in an excess amount relative to the estimated amount or concentration of suspected analyte in the test medium. Such excess amount provides a sufficient number of specific binding partners for the suspected analyte in the test medium so that substantially all of the suspected analyte becomes bound by the labeled reagent. It is to be appreciated that since the excess labeled reagent binds and thereby renders substantially all of the suspected analyte in the test medium detectable, an accurate and highly sensitive determination of the analyte can be made.

The use of excess amounts of reagents as heretofore described results in favorable kinetic and/or equilibrium advantages. In particular, excess amounts of reagents accelerate the kinetics of the binding reactions and shifts the equilibrium of the reaction to favor the formation of the bound species, even at low analyte concentrations, whereby the limiting reagent of the assay becomes the analyte itself. Accordingly, since the assay reagents are in excess amounts, the effect of variations in the concentration thereof on assay performance is less significant than, for example, that which is encountered in a conventional competitive immunoassay format, and a margin of error in reagent additions is thereby permitted when made at least in excess amounts.

Furthermore, by shifting the equilibrium as heretofore described, it also becomes possible to maximize the amount of analyte bound by the labeled reagent and therefore optimize the sensitivity of the assay. Still further, where the single antibody immunometric method of the present invention is employed, the presence of nonspecific binding does not create a background signal which would reduce the sensitivity of the assay, such as that encountered in conventional sandwich immunoassays where two antibody binding reactions are involved and wherein the label bound to a solid phase material or reagent particle is measured. The single antibody immunometric method also permits the application thereof to the detection of analytes having a single epitope, which is not feasible in a sandwich immunoassay. Furthermore, where monoclonal monovalent antibodies are employed in excess amounts in an assay, together with excess amounts of other assay reagents, the production of a linear dose response curve which facilitates the application of such assay format to a convenient calibration procedure is provided.

It is to be understood that although the aforementioned assay reagents can be combined simultaneously to form the reaction mixture as heretofore described, the assay reagents are preferably combined sequentially to form the reaction mixture. In particular, a first reaction mixture comprising the test medium containing the analyte and the labeled reagent is formed and incubated for a predetermined period of time sufficient to permit binding of all of the analyte from the test medium to the labeled reagent. The first reaction mixture is then combined with the reagent particles to form a second reaction mixture and incubated for a predetermined period of time to permit binding of all of the free species of the labeled reagent to the reagent particles.

Once the reaction mixture has been formed and the appropriate binding reactions have occurred as heretofore described, an aliquot of the reaction mixture is then applied to the separation zone, or the separation zone of the test device is immersed into the reaction mixture. As will be described in greater detail hereinafter, the separation of the free species of the labeled reagent bound to the reagent particle from the bound species of the labeled reagent is achieved by the ability of the separation zone to retain or otherwise immobilize the reagent particle. Such immobilization of the reagent particle thereby prevents the migration thereof into the detection zone while, at the same time, permits the capillary transport of the bound species out of the separation zone and into the detection zone to effect such separation. Accordingly, the ability of the separation zone to retain the reagent particle will therefore depend upon an interaction between the reagent particle and the separation zone matrix material whereby the separation zone is rendered substantially impervious thereto as a result of such interaction.

It is to be understood that such interaction between the reagent particle and the separation zone matrix material can be any interaction which will render the separation zone matrix substantially impervious to the reagent particle. For example, the separation zone matrix material and the reagent particle can be biologically modified with binding counterparts, such as biotin and avidin or other specific binding partners, and the like, to possess an interactive binding property whereby the reagent particle binds to and is immobilized by the separation zone matrix material upon contact therewith. Alternatively, the separation zone matrix material and/or the reagent particle can be chemically modified to possess a desired binding interaction therebetween, such as ionic, hydrophobic, covalent, and the like, binding interactions.

Preferably, the reagent particle is retained by the separation zone matrix material by filtration whereby the reagent particle is a size which is sufficient to infiltrate the matrix material of the separation zone while, at the same time, is precluded by the matrix material from migrating therein to any significant degree. In particular, the reagent particles are generally slightly larger than the minimum effective pore size of the separation zone matrix material and generally smaller than the maximum effective pore size thereof, which will be described in greater detail hereinafter.

Reagent Particle

The reagent particle employed by the method of the present invention comprises a water insoluble particle to which the analyte under determination, or a binding analog thereof, is capable of being covalently bound, noncovalently bound (e.g., physical absorption), or otherwise immobilized thereto. Although the particle is generally in the form of a bead, other configurations may be employed as well which would be compatible with the teachings of the present invention. For example, such configurations include rods, fibers, geometric configurations, and the like, and irregular configurations thereof. Generally, such particles are microparticles having as a largest dimension thereof, less than 50 microns, preferably from between about 0.1 microns and 20 microns, more preferably, from between about 0.5 microns and 3.0 microns.

In particular, such particles can be made from materials such as polystyrene, polyacrylates, polyacrylamide, or naturally occurring materials such as polysaccharides, particularly cross-linked polysaccharides, such as agarose, dextran, microcrystalline cellulose, starch, fibrous particles such as glass fibers and cellulose fibers, and the like. Other materials include polyvinyltoluene, or styrenebutadiamine copolymers, polyacrolein microspheres, polyurethane, pollen particles, sporopollenin, polystyrene or polyvinylnapthalene cores surrounded by shells of polyclycidyl methacrylate, microcrystalline cellulose or combinations thereof, polyvinyl alcohol, copolymers of hydroxyethyl methacrylate and methyl methacrylate, silicones and silica, glass, rubber, nylon, diatomaceous earth, silica, and the like.

Preferably, the particles employed according to the present invention are uniform, water suspensible, latex particles. Such particles are commercially available or can be prepared by emulsion polymerization, or by suspension polymerization, resulting in particle sizes of less than 5 microns in diameter, or greater than 5 microns in diameter, respectively. In addition, particle sizes in the range from 2-20 microns in diameter can be effectively produced by "swollen emulsion polymerization" (Bangs, L., *Uniform Latex Particles*, Seragen, Inc., 1984).

It is to be understood that latex particles encompass any one of a wide variety of particulate materials which are dispersable or suspendable in aqueous media without significant gravitational settling and therefore capable of remaining in suspension in the reaction mixture without constant mixing. Accordingly, together with Brownian motion and the high surface to volume ratio, efficient and rapid binding kinetics are assured.

Where it is desirable to covalently bind the analyte or binding analog thereof to the particle, the particle should be polyfunctional or capable of being polyfunctionalized. Similarly, a wide variety of latex particles having such functional groups are commercially available, or can be incorporated according to covalent coupling techniques known in the art [see, for example, Cuatrecasas, *J. Biol, Chem.*, Vol. 245, p. 3059-(1970)]. Functional groups include carboxylic acids, aldehydes, amines, amides, activated ethylenes such as maleimide, hydroxyls, sulfonic acids, mercaptans, and the like. For example, coupling of analytes and other biological molecules to agarose and polyacrylamides are described by Cuatrecasas in *J. Biol. Chem.* *245*, p. 3059-3065 (1970) and W. B. Jacoby and M. Wilchek, *Methods in Enzymology*, Vol. 34, Academic Press, New York (1974).

The analyte or binding analog thereof immobilized to the particle as heretofore described can be selected for the determination of any analyte for which there is a binding counterpart available. The analyte usually is a peptide, polypeptide, protein, carbohydrate, glycoprotein, steroid, nucleic acid or other organic molecule for which a binding counterpart exists or which is producible in biological systems or can be synthesized. The analyte, in functional terms, is usually selected from the group comprising antigens, haptens, complementary polynucleotide sequences, hormones, vitamins, metabolites and pharmacological agents. Usually, the analyte is an immunologically-active polypeptide or protein, usually having a molecular weight of between about 1,000 and about 10,000,000, such as an antigenic polypeptide or protein, or a hapten having a molecular weight of at least about 100, and usually less than about 1,500.

Representative polypeptide analytes are angiotensin I and II, C-peptide, oxytocin, vasopressin, neurophysin, gastrin, secretin, bradykinin, and glucagon.

Representative protein analytes include the classes of protamines, mucoproteins, glycoproteins, globulins, albumins, scleroproteins, phosphoproteins, histones, lipoproteins, chromoproteins, and nucleoproteins. Examples of specific proteins are prealbumin, $\alpha_1$-lipoproteins, human serum albumin, $\alpha_1$-acid glycoprotein, $\alpha_1$-antitrypsin, $\alpha_1$-glycoprotein, transcortin, thyroxine binding globulin, haptoglobin, hemoglobin, glycosylated peptide sequences such as the glucosylated N-terminal peptide sequence in the beta-subunit of human hemoglobin, myoglobulin, ceruloplasmin, $\alpha_2$-macroglobulin, $\beta$-lipoprotein, erythropoietin, transferrin, hemopexin, fibrinogen, the immunoglobulins such as IgG, IgM, IgA, IgD, and IgE, and their fragments, e.g., $F_c$ and $F_{ab}'$ complement factors, prolactin, blood clotting factors such as fibrinogen, thrombin and so forth, insulin, melanotropin, somatotropin, thyrotropin, follicle stimulating hormone, leutinizing hormone, gonadotropin, human chorionic gonadotropin thyroid stimulating hormone, placental lactogen, instrinsic factor, transcobalamin, serum enzymes such as alkaline phosphatase, lactic dehydrogenase, amylase, lipase, phosphatases, cholinesterase, glutamic oxaloacetic transaminase, glutamic pyruvic transaminase, and uropepsin, endorphins, enkephalins, protamine, tissue antigens, bacterial antigens, viral antigens such as hepatitis associated antigens (e.g., $HB_sAg$, $HB_cAg$ and $HB_eAg$), and tumor markers (e.g., CEA, alpha fetoprotein, prostatic acid phosphatase, prostatic specific antigen, neuron specific enolase, estrogen receptor, CA125, CA19-9, and the like).

Representative hapten analytes include the general classes of drugs, metabolites, hormones, vitamins, toxins and the like organic compounds. Haptenic hormones include thyroxine and triiodothyronine. Vitamins include vitamins A, B, e.g., thiamine, $B_{12}$, C, D, E and K, and folic acid. Drugs include antibiotics such as aminoglycosides, e.g., gentamicin, tobramycin, amikacin, sisomicin, kanamycin, and netilmicin, penicillin, tetracycline, terramycine, chloromycetin, and actinomycetin; nucleosides and nucleotides such as adenosine diphosphate (ADP) adenosine triphosphate (ATP), flavin mononucleotide (FMN), nicotinamide adenine dinucleotide (NAD) and its phosphate derivative (NADP), thymidine, guanosine and adenosine; prostaglandins; steroids such as the estrogens, e.g., estriol and estradiol, sterogens, androgens, digoxin, digitoxigenin, digitoxin, digoxigenin, 12-0-acetyldigoxigenin, and adrenocortical steroids; and others such as phenobarbital, phenytoin, primidone, ethosuximide, carbamazepine, valproate, theophylline, caffeine, propranolol, procainamide, quinidine, amitryptiline, cortisol, desipramine, disopyramide, doxepin, doxorubicin, nortryptiline, methotrexate, imipramine, lidocaine, procainamide, N-acetylprocainamide, amphetamines, catecholamines, and antihistamines. Toxins include acetyl T-2 toxin, alfatoxin, cholera toxin, citrinin, cytochalasins, staphylococcal enterotoxin B, HT-2 toxin, and the like.

## Labeled Reagent

The labeled reagent employed according to the present invention comprises a specific binding partner for the analyte under determination labeled with a detectable chemical group. The specific binding partner is one which is capable of binding to either the analyte from the liquid test medium or to the analyte or binding analog thereof immobilized to the reagent particle.

The specific binding partner for the analyte can be a whole antibody, such as any of the classes and subclasses of known immunoglobulins, e.g., IgG, IgM, and the like, or monovalent and divalent antibody fragments of IgG, conventionally known as Fab and Fab', and F(ab)$_2$, respectively. The immunoglobulin source for the antibody component can be obtained in any available manner such as conventional antiserum and monoclonal techniques. Antiserum can be obtained by well-established techniques involving immunization of an animal, such as a mouse, rabbit, guinea pig or goat, with an appropriate immunogen. The immunoglobulins can also be obtained by somatic cell hybridization techniques, such resulting in what are commonly referred to as monoclonal antibodies, also involving the use of an appropriate immunogen.

Preferably, the antibody will commonly be a divalent antibody fragment [F(ab′)₂] or, more preferably, a monovalent antibody fragment (Fab or Fab′). Divalent and monovalent IgG antibody fragments can be obtained according to methods known in the art employing standard proteolytic digestion procedures with pepsin and papain. For example, divalent antibody fragments [F(ab′)₂] can be prepared by digesting the whole IgG antibody with pepsin [Nisonoff, *Methods Med. Res.*, Vol. 10, 132(1964)], and then reducing the F(ab′)₂ fragment to the corresponding Fab′ fragment with dithiothreitol. Alternatively, Fab fragments can be obtained by papain digestion of IgG [Porter, *Biochem. J.,* Vol. 73, 119(1959)].

The detectable chemical group of the labeled reagent can be any material having a detectable physical or chemical property. Such materials have been well-developed in the field of immunoassays and in general any label useful in such methods can be applied to the present invention.

In particular, such chemical groups having detectable physical properties are those groups which are detected on the basis of their own physical properties which do not require a chemical reaction or interaction with another chemical or substance to provide a detectable signal. Such groups principally include fluorescers such as umbelliferone, fluorescein, resorufin, various rhodamines, dansyl derivatives and aminoanaphthalenesulfonic acid, [see *Clin. Chem.*, 25:353(1979)]; phosphorescent molecules such as pyrene, 4-nitrobiophenyl, benzaldehyde, benzophenone or the trivalent metal chelates of dibenzoylmethane (e.g., Al$^{+3}$, T$^{+3}$); chromophores such as para- or ortho-nitrophenol, phenolphthalein, napthol AS, para-nitroanilide and thymolphthalein; radioisotopes such as $^3$H, $^{35}$S, $^{32}$P, $^{125}$I and $^{14}$C; spin labels including nitroxide radicals such as DOXYL, PROXYL and TEMPO derivatives; or electroactive moieties such as protons, fluoride, oxygen, ammonia and hydrogen peroxide.

Chemical groups having detectable chemical properties are those groups which are detected on the basis of their own chemical reactivity or interaction with a detectant component therefor to provide a detectable signal. Such chemical groups having detectable chemical properties do not generate a detectable product or otherwise provide a detectable signal prior to interacting with such detectant component and include enzymatically active groups such as enzymes (see *Clin. Chem.* 22:1232 (1976), U.S. Reissue Pat. 31,006, and U.K. Pat. 2,019,308), enzyme substrates (see British Pat. Spec. 1,548,741), coenzymes (see U.S. Pat. Nos. 4,230,797 and 4,238,565), enzyme inhibitors and activators, chemiluminescent species, chemical catalysts, metal catalysts, members of enzyme channeling, fluorophor-quencher, or energy transfer pairs (see U.S. Pat. Nos. 3,996,345; 4,174,384; 4,199,559, and 4,233,401), and specifically bindable ligands such as biotin or a hapten.

Where the label of the labeled reagent is a detectable chemical group having a detectable chemical property, the detectant component therefore is incorporated into the detection zone of the reagent particle separation and detection test device of the present invention. Preferably, such detectable chemical group is an enzyme, whereby the particular substrate for the selected enzyme label is incorporated into the detection zone of the test device. Particular enzymes which can be used include, but are not intended to be limited to, β-D-galactosidase, peroxidase, glucose oxidase, alkaline phosphatase, glucose-6-phosphate dehydrogenase, luciferase, pyruvate kinase, lactate dehydrogenase, galactose oxidase, tyrosinase, invertase, xanthanine oxidase, urease, uricase, alcohol oxidase, and the like.


Reagent Particle Separation and Detection Test Device


The reagent particle separation and detection test device comprises a solid, porous matrix which is capable of transporting aqueous liquids and comprises a separation zone and a detection zone which is situated a predetermined distance away from and in liquid transport contact with the separation zone. As will be described in greater detail hereinafter, the separation zone and the detection zone can be composed of and located along the length of a single, continuous matrix material, or can be composed of physically distinct matrix materials in liquid flow contact.

In particular, the separation zone comprises a solid, porous matrix material, preferably fibrous, which is substantially impervious to the reagent particle as described above, and is permeable to aqueous liquids and at least some components of a liquid test sample, e.g., antigens, haptens, antibodies, and the like. It is to be understood that the liquid test medium containing the analyte under determination can be a naturally occurring or artifically formed liquid suspected to contain analyte, and is usually a biological fluid or a dilution thereof. Such biological fluids include serum, whole blood, plasma, urine, saliva, and amniotic and cerebrospinal fluids. Accordingly, in most instances, selection of a matrix material which is substantially impervious to the reagent particle will also be one which is capable of removing cellular components such as red blood cells, white blood cells, platelets, and the like, from such biological fluids.

Preferably, the separation zone matrix material is selected from various fibrous materials known in the art which are capable of filtering reagent particles and which are capable of transporting aqueous liquids by capillary action. More preferably, the separation zone matrix material should be capable of rapid capillarity in order to concentrate the reagent particles at the site of contact with the liquid reaction mixture. Such matrix materials include, but are not intended to be limited to, paper, glass fiber, porous plastic, scintered glass or plastic, cellulose particles, gels of agarose or other polymers, or woven materials such as cloth, nylon or other polymeric mesh material, or other fibrous materials, whether formed from natural materials or synthetic materials. For example, U.S. Patent No. 3,846,247 describes the use of felt and woven or matted glass fibers, and the use of synthetic resin fleeces and glass fiber felts is described in Great Britain Patent No. 1,369,139. Other fibrous matrix materials are also described in U.S. Patent Nos. 3,802,842, 3,809,605, 3,897,214, and 3,987,214.

The various matrix materials employed according to the present invention can also be treated with substances known in the art to facilitate the permeability of the bound species of the labeled reagent. For example, detergents such as Tween 20®, Triton X-100®, or sodiumdodecylsulfate can be added to either the matrix materials or to the liquid test sample or reaction mixture. Similarly, protein such as bovine serum albumin, lactalbumin, or gamma globulin, can be added to the liquid test sample, the test device, or to the reaction mixture.

As will be understood by one skilled in the art, the ability of the separation zone matrix material to filter the reagent particle will depend upon the size or shape of the reagent particle and the effective pore size or shape of the particular material from which the separation zone is made. In particular, the matrix material of the separation zone comprises a minimum pore size which is at least slightly smaller than the size of the particular latex particle selected whereby upon contact with the separation zone, the reagent particle is precluded from migrating out of the separation zone and into the detection zone. It is to be understood that in order for the reagent particle to be entrapped by the separation zone matrix, the matrix material preferably possesses a maximum effective pore size as well, to a limited degree, which is larger than the latex particle to permit the infiltration of the latex particle as described above. Accordingly, the selection of an appropriate separation zone matrix material can be made by one skilled in the art apprised of the foregoing considerations.

Where the separation zone and the detection zone are composed of a single, continuous matrix material as heretofore described, the detection zone will be, of course, composed of the same matrix material and possess the same characteristics as the separation zone matrix material. It will be understood, however, that where it is desirable for the separation zone and the detection zone to be composed of physically distinct matrix materials, such materials can be the same or different, whereby the detection zone matrix material can be selected from the aforementioned matrix materials. Where such matrix materials are different, the detection zone matrix material must at least be capable of transporting aqueous liquids by capillary action, but does not necessarily have to be substantially impervous to the reagent particle as does the separation zone matrix material.

According to the present invention, the detection zone receives the bound species of the labeled reagent which migrates out of the separation zone and into the detection zone by capillary action. Once in the detection zone, the detectable signal provided by the label of the bound species of the labeled reagent is measured and correlated to the amount of analyte present in the liquid test sample. The generation of the detectable signal will of course depend upon the particular label employed, i.e., that which possesses either a detectable physical property or a detectable chemical property. For example, where the label possesses a detectable physical property, the label of the bound species inherently provides a detectable signal which, once in the detection zone, can be readily measured without interacting with a detectant component therefor to provide the detectable signal in the detection zone. On the other hand, where the label possesses a detectable chemical property, the detection zone is incorporated with the detectant component for the label, such as an enzyme substrate where the label is an enzyme, which interacts with the label to provide the detectable signal in the detection zone. In either case, the signal generated by the label of the bound species in the detection zone is measured with an appropriate instrument known in the art, such as a spectrophotometer, particularly a Seralyzer® reflectance photometer (Miles Diagnostics, Elkhart, IN, USA) with which the test device of the present invention is readily adaptable.

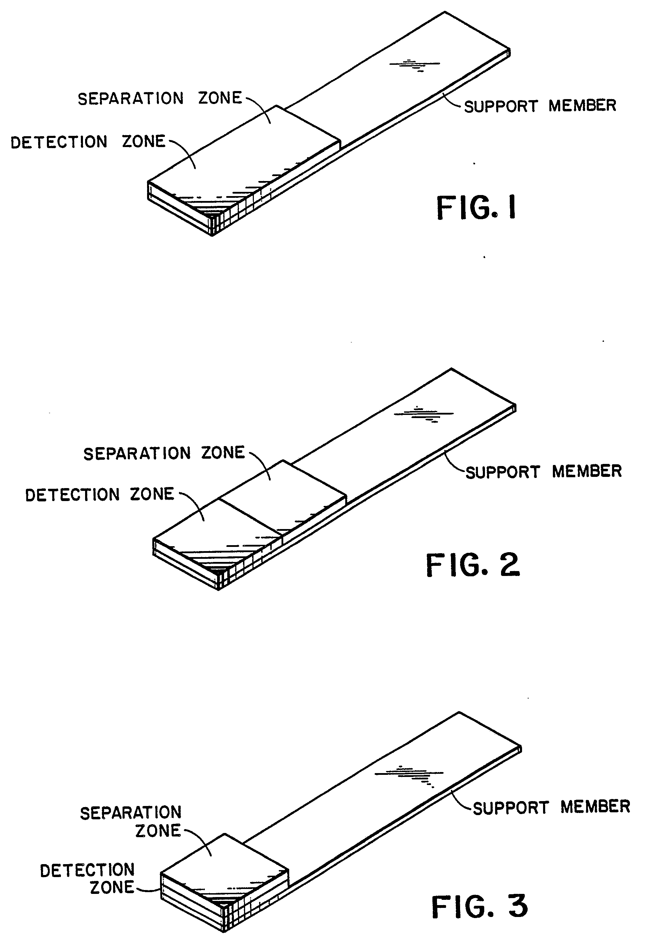

According to one preferred embodiment of the present invention (Fig. 1), the separation zone and the detection zone are located at physically distinct portions along the length of an elongate strip composed of a single, continuous matrix material which is substantially impervious to the reagent particle as heretofore described. The separation zone and detection zone are situated a predetermined distance away from and in liquid transport contact with each other along the length of the strip to permit the separation of the reagent particle at one end of the strip, and the detection of the bound species of the labeled reagent at the other end of the strip, respectively.

It is to be understood that since the separation of the reagent particle from the bound species of the labeled reagent arises from the ability of the matrix material to transport aqueous liquids by capillary action, such separation is therefore best achieved by transporting a relatively large volume of the liquid test medium through a relatively narrow area. Accordingly, the width of the elongate strip is generally, but not intended to be limited to, less than 20 mm wide, preferably from between about 1.0 mm and 12 mm wide, more preferably from between about 4.0 mm to 8.0 mm wide. The length of the strip is generally, but not intended to be limited to, from between about 2.0 cm and 40 cm long, preferably from between about 4.0 cm to 25 cm long, more preferably from between about 6.0 cm to 20 cm long.

According to another preferred embodiment of the present invention (Fig. 2), the separation zone and the detection zone are composed of, individually, physically distinct portions similarly located along the length of an elongate strip and composed of physically distinct matrix materials. In particular, the individual matrix materials, which can be the same or different as heretofore described, are positioned in an end-to-end relationship whereby the ends thereof are in liquid transport contact to permit the migration of the bound species of the labeled reagent into the detection zone to effect the separation thereof from the reagent particle as heretofore described. It is to be understood that although the individual matrix materials preferably form an elongate strip having dimensions similar to those described above, the individual matrix materials forming the separation zone and the detection zone can vary in length whereby the length of one can be different from, and not necessarily the same as, the length of the other.

According to still another preferred embodiment of the present invention (Fig. 3), the separation zone and the detection zone are composed of physically distinct matrix materials, which can be the same or different, mounted or otherwise positioned relative to each other in the form of layers in liquid transport contact. Such layers can be maintained in a laminar relationship by adhesives known in the art which permit aqueous fluid passage and components thereof as heretofore described between the layers to permit the separation of the reagent particle from the bound species. Although the respective layers are generally of equal length and width, i.e., square, other compatible configurations can be selected by one skilled in the art.

Where a layered test device as described above is employed with a labeled reagent comprising a detectable chemical group having a detectable physical property, it is desirable to further include a radiation-blocking zone or layer positioned between the separation layer and the detection layer. By incorporating such layer between the separation layer and the detection layer, any signal produced from the bound species in the detection layer would be detected without an interfering signal produced by the labeled reagent bound to the reagent particle in the separation layer as a result of such non-transmissive layer incorporated therebetween. In this manner, the signal produced from the detection layer can be detected, measured, and correlated to the amount of analyte in the liquid test medium without an interfering signal produced from the separation layer.

Alternatively, it may be desirable to utilize radiation-blocking agents which would be incorporated into the separation layer. Opacifying pigments, such as titanium dioxide, barium sulfate or zinc oxide can be used for this purpose. Blush polymers can also be used, either independently, or incorporated with pigment to enhance radiation-blocking or other properties. Such radiation-blocking layers and agents are known in the art and include those described in U.S. Pat. Nos. 4,042,335 and 4,255,384.

It is to be understood that the thickness of the separation zone and detection zone matrix materials as heretofore described, as well as the degree of permeability thereof, will vary and depend upon actual usage. Generally, a dry thickness from between about 0.1 mm and 2.0 mm is preferable, although more widely varying thicknesses can be selected by one skilled in the art apprised of the foregoing considerations and depending upon the particular circumstances of usage. Furthermore, the separation zone and detection zone matrix materials are preferably mounted or otherwise positioned onto a solid, nonporous support member with an appropriate adhesive. A wide variety of organic and inorganic materials, both natural and synthetic, and combinations thereof, may be employed provided only that the support does not interfere with the capillary action of the strip, or non-specifically bind assay components, or interfere with the signal producing system. Illustrative polymers include polyethylene, polypropylene, poly(4-methylbutene), polystyrene, polymethacrylate, poly(ethylene terephthalate), nylon, poly(vinyl butyrate), glass, ceramics, metals, and the like.

## Test Kit

The present invention further provides a test kit comprising all of the essential elements required to conduct the immunoassay method of the present invention. The test kit is presented in a commercially packaged form, as a composition or admixture where the compatability of the reagents will allow, in a test device configuration, or more usually as a test kit, i.e., a packaged combination of one or more containers, devices or the like holding the necessary reagents, and usually including written instructions for the performance of the immunoassay.

In particular, the test kit will at least comprise (1) reagent particles comprising the analyte or a binding analog thereof immobilized to insoluble microparticles, preferably latex particles, (2) a labeled reagent, comprising a labeled form of a specific binding partner for the analyte, preferably a monovalent antibody fragment derived from a monoclonal antibody to the analyte under determination, labeled with an enzyme, and (3) the reagent particle separation and detection test device of the present invention, preferably incorporated with a substrate for the.enzyme, which generates a detectable signal which can be measured and correlated to the amount of analyte present in a liquid test sample.

In particular, the present invention will now be illustrated, but is not intended to be limited, by the following examples:

## EXAMPLE 1

### Separation of Latex Particles on a Paper Matrix Material

(a) In order to demonstrate the ability of a paper matrix material to separate latex particles from an aqueous liquid test medium, a 15.2 cm x 5.1 cm sheet of Whatman 54 paper (Whatman, Ltd., Maidstone, England) was impregnated with 5.0 mM $MgCl_2$ in 0.1 M bicine (pH 8.3), air dried, and then cut into 2.0 cm x 0.80 cm elongate strips. A 20.0 $\mu l$ aliquot of a 2.5% (weight/volume) aqueous suspension of 3.0 $\mu m$ diameter red polystyrene latex particles (Polybead®, Polysciences, Inc., Warrington, PA, USA) in water was pipetted onto a centrally located area of a strip at approximately 0,4 cm from one of the ends thereof identified as the separation zone, the other end thereof being identified as the detection zone. Although both the separation zone and the detection zone were wetted by the water of the suspension, the latex particles were visually observed only at the site of application thereof, i.e., the separation zone, and did not migrate into the detection zone.

(b) In order to demonstrate the ability of particles having a diameter sufficiently less than the minimum effective pore size of the matrix material to migrate therethrough, the aforementioned latex particle suspension was prepared employing 1.0 $\mu m$ diameter red latex particles and applied to another elongate strip as described above. In this case, the latex particles were visually observed in the detection zone to indicate that the particles were not retained by the paper matrix material.

## EXAMPLE 2

### Separation of Latex Particles on a Glass Fiber Matrix Material

In order to demonstrate the ability of a glass fiber matrix material to separate latex particles from an aqueous liquid test medium, a 15.2 cm x 5.1 cm sheet of Whatman GFA glass fiber (Whatman, Ltd., Maidstone, England) was cut into 0.5 cm x 2.0 cm elongate strips and mounted onto one end of 0.5 cm x 8.0 cm elongate strips (0.04 cm thick) of polystyrene (Trycite®, Dow Chemical Co., Midland, MI, USA) with double-faced adhesive tape (Part No. 2976MR, 3M Company, St. Paul, MN, USA). One end thereof was identified as the separation zone and the other end thereof was identified as the detection zone.

Polystyrene latex particles (Polybead®, Polysciences, Inc., Warrington, PA, USA) having mean diameters of 0.48 $\mu$m, 0.83 $\mu$m and 3.0 $\mu$m were derivatized with antibody protein and $^{125}$I labeled protein A by first forming 2.5% suspensions thereof (weight/volume) in 0.1 M Tris-Cl pH 7.0 with 5.0 mg/mL bovine gamma globulin (fraction II, Sigma, St. Louis, MO, USA). The particles were incubated at 25°C for 30 minutes with sonication. The gamma globulin solution was removed and the particles were washed four times with a wash buffer comprising 0.1% bovine serum albumin, 0.1% Tween 20, and 50 mM Tris-Cl (pH 7.0). The particles were resuspended to 2.5% (weight/volume) in the wash buffer and 20.0 $\mu$L of 2.0 $\mu$Ci $^{125}$I-protein A (30.0 $\mu$g/mL, E. I. duPont de Nemours & Co., Inc., NEN Research Products, Boston, MA, USA) was added per milliliter of latex suspension. The latex suspensions were incubated with $^{125}$I-protein A for one hour at room temperature and decanted. They were then washed three times with the wash fluid described above and the $^{125}$I-protein A coated latex particles were stored and used at 2.5% (weight/volume) in the wash buffer.

Each of the sized $^{125}$I-protein A coated latex particle preparations were tested three times on the glass fiber elongate strips described above. In this procedure, 30.0 $\mu$L of the suspension were pipetted onto the separation zone of a glass fiber elongate strip. After thirty seconds, the elongate strip was cut in half and $^{125}$I-disintegrations from each half measured for one minute in a gamma counter. The percentage of $^{125}$I-protein A labeled particles that remained in the separation zone of the elongate strip was determined for each sample as counts per minute (cpm) in the separation zone divided by, cpm in the separation zone plus cpm in the detection zone, as set forth in Table 1.

Table 1

| Particle Size | Average % Retention in Separation Zone |
|---|---|
| 0.48 $\mu$m diameter | 99.3% |
| 0.83 $\mu$m diameter | 99.8% |
| 1.3 $\mu$m diameter | 99.9% |
| 3.0 $\mu$m diameter | 99.8% |

EXAMPLE 3

Preparation of Reagent Particle Separation and Detection Test Device and Use Thereof in an Immunoassay

(a) Test Device

A 3.81 cm x 15.24 cm sheet of Whatman GFA glass fiber was impregnated by immersion into 10.0 mM resorufin-galactoside (prepared according to the method described by European Patent Application Publication No. 156,347) and 1.0% polystyrene in dimethylformamide, and dried by forced air at 50°C for 10 minutes. Double-faced adhesive tape was applied to one side of the impregnated glass fiber sheet, and then cut into 2.0 cm wide elongate strips. The elongate strips were then laminated at one end of 8.3 cm x 2.0 cm polystyrene sheets (0.4 cm thick), and then cut into 8.3 cm x 0.5 cm elongate strips. The elongate strips were stored in brown glass bottles with a desiccant at room temperature.

(b) Reagent Particles

3-Hydroxy-(5-carboxypentyl)-carbamoyl digitoxigenin (0.132 mmole) was added to 0.132 mmole isobutylchloroformate and 0.132 mmole triethylamine in 5.0 mL dimethylformamide at -10° to -20° C under argon, and then added dropwise to a solution of bovine serum albumin (110.0 mg/10.0 mL) in 0.1 L pyrophosphate (pH 8.5) with vigorous stirring at 0-4° C for a period of 15 minutes. The reaction mixture was then sonicated for one hour. After storage at 4° C overnight, the reaction mixture was adjusted to pH 7.0, loaded onto a 45.0 cm x 2.5 cm Sephadex® column (Sigma, St. Louis, MO, USA) equilibrated with 25.0 mM sodium phosphate buffer (pH 7.0), and eluted with 25.0 mM sodium phosphate buffer (pH 7.0).

Carboxylate modified polystyrene latex particles (1.3 um average diameter, 0.02-0.05 mequiv. of carboxylate/g particles; Seragen, Inc., Indianapolis, IN, USA) were rinsed twice with water and resuspended at 5.0% (weight/volume) in 0.1 M borate buffer (pH 8.5) which contained 0.05 mg/mL of the BSA-digitoxigenin reagent described above. The suspension was incubated for 20 hours and washed three times with 20.0 mM sodium phosphate, 0.1% Tween 20 (pH 7.0), twice in 1.0% sodiumdodecylsulfate for 30 minutes at 60° C, once in 20.0 mM sodium phosphate, 0.1% Tween 20 (pH 7.0), and twice in ethanol. The particles were stored at 5.0% (weight/volume) in ethanol at 4° C. The ethanol was decanted and the latex particles resuspended to 1.0% (weight/volume) in reagent buffer (0.05 M sodium phosphate, 0.05 M NaCl, 1.0 mM $MgCl_2$, 0.02% $NaN_3$ and 0.01% bovine serum albumin, pH 7.4) before use.

(c) Immunoassay and Operation of the Test Device

An immunoassay reaction mixture was formed comprising digoxin calibrator serum (Optimate® liquid analyzer, Miles Diagnostics, Elkhart, IN, USA) diluted five-fold into the reagent buffer described above containing a labeled reagent (1.0 nM of a substantially pure anti-digoxin/$\beta$-D-galactosidase monoconjugate preparation comprising a single monovalent antibody fragment derived from an anti-digoxin monoclonal antibody coupled to a single $\beta$-D-galactosidase molecule). After 5 minutes at room temperature, an equal volume of 2.0% (weight/volume) of the latex reagent was added and incubated for 3 minutes at room temperature.

An elongate strip as described above was placed onto the strip holder of a Seralyzer® reflectance photometer (Miles Diagnostics, Elkhart, IN, USA) and 30.0 $\mu$l of the reaction mixture described above was applied to the separation zone of the elongate strip. The signal generated by the labeled reagent bound to the serum digoxin which migrated into the detection zone was measured [K/S rate signal response

$$= \frac{(1 - \text{reflectance})^2}{(2 \times \text{reflectance})},$$

determined between 70-90 seconds after placing an aliquot on the elongate strip] as set forth in Table 2.

Table 2

| SERUM DIGOXIN DOSE RESPONSE | |
|---|---|
| ng/ml Serum Digoxin | K/S Rate x $10^{-2}$ |
| 0 | 4.4 |
| 0.6 | 5.6 |
| 1.3 | 8.2 |
| 2.5 | 9.8 |
| 5.0 | 13.0 |

# EP 0 297 292 A2

**Claims**

1. A method for specific binding assay determination of an analyte in an aqueous liquid test medium employing (i) a reagent particle comprising the analyte or a binding analog thereof immobilized to an insoluble particle, (ii) a labeled reagent comprising a specific binding partner for the analyte labeled with a detectable chemical group, and (iii) a reagent particle separation and detection test device comprising a solid, porous matrix which is capable of transporting aqueous liquids and which comprises a separation zone which is substantially impervious to said reagent particle and a detection zone which is situated a predetermined distance away from and in liquid transport contact with said separation zone, said method characterized by the steps of:

(a) forming an aqueous liquid reaction mixture comprising said test medium, said labeled reagent, and said reagent particle in an amount in excess of that capable of binding to all of said labeled reagent which does not become bound to said analyte;

(b) applying said reaction mixture to said separation zone of said test device, whereby said reagent particle is retained in said separation zone and said labeled reagent not bound to said reagent particle is transported into said detection zone by said liquid medium; and

(c) measuring said detectable chemical group of said labeled reagent in said detection zone of said test device.

2. The method of claim 1 characterized in that said aqueous liquid reaction mixture is formed by (i) forming a first reaction mixture comprising said test medium and said labeled reagent; (ii) incubating said first reaction mixture for a predetermined period of time; (iii) forming a second reaction mixture comprising said first reaction mixture and said reagent particle; and (iv) incubating said second reaction mixture for a predetermined period of time.

3. The method of claim 1 or 2 characterized in that said insoluble particle is a water suspensible latex particle.

4. The method of any one of claims 1 to 3 characterized in that said test device comprises an elongate strip composed of a solid, porous matrix material capable of transporting aqueous liquids along the length thereof by capillary action, said separation and detection zones being located at physically distinct portions along said strip.

5. The method of claim 4 characterized in that said elongate strip is composed of a single, continuous matrix material which is substantially impervious to said reagent particle.

6. The method of claim 4 or 5 characterized in that said matrix material is a fibrous material, preferably paper, glass, cellulose or a woven fabric.

7. The method of any one of claims 4 to 6 characterized in that said physically distinct portions of said strip comprising said separation and detection zones are composed of physically distinct matrix materials in liquid transport contact.

8. The method of any one of claims I to 7 characterized in that said detectable chemical group possesses a detectable chemical property and interacts with a detectant component to provide a detectable signal, and said detection zone is incorporated with said detectant component.

9. A reagent particle separation and detection test device for use in the determination of an analyte in an aqueous liquid test sample wherein a specific binding assay reaction mixture is formed involving binding among (i) the analyte, (ii) a reagent particle comprising the analyte or a binding analog thereof immobilized to an insoluble particle, and (iii) a labeled reagent comprising a specific binding partner for the analyte labeled with a detectable chemical group characterized in that said test device comprises:

a solid, porous matrix which is capable of transporting aqueous liquids and which comprises a separation zone which is substantially impervious to said reagent particle and a detection zone which is situated a predetermined distance away from and in liquid transport contact with said separation zone.

10. A test kit for the specific binding assay determination of an analyte in an aqueous liquid test sample characterized in that said test kit comprises:

(a) a reagent particle comprising the analyte or a binding analog thereof immobilized to an insoluble particle;

(b) a labeled reagent comprising a specific binding partner for the analyte labeled with a detectable chemical group; and

(c) a reagent particle separation and detection test device comprising a solid, porous matrix which is capable of transporting aqueous liquids and which comprises a separation zone which is substantially impervious to said reagent particle and a detection zone which is situated a predetermined distance away from and in liquid transport contact with said separation zone.

14

SEPARATION ZONE

DETECTION ZONE

SUPPORT MEMBER

FIG. I

SEPARATION ZONE

DETECTION ZONE

SUPPORT MEMBER

FIG. 2

SEPARATION ZONE

DETECTION ZONE

SUPPORT MEMBER

FIG. 3